# FASCICULE DE BREVET EUROPEEN

(11) **EP 0 496 446 B1**
(45) Date de publication et mention de la délivrance du brevet: **17.09.1997**
(21) Numéro de dépôt: 92200086.4
(22) Date de dépôt: 14.01.1992
(51) Int. Cl.: C07C 21/18, C07C 17/00, B01J 23/89

(54) **Procédé pour la préparation de chlorotrifluoroéthylène et de trifluoroéthylène au départ de 1,1,2-trichloro-1,2,2-trifluoroéthane et composition catalytique utilisée dans ce procédé**
Verfahren zur Herstellung von Chlortrifluorethylen und von Trifluorethylen aus 1,1,2-trichlor-1,2,2-trifluorethan sowie katalytische Zusammensetzung zur Verwendung in diesem Verfahren
Process for the preparation of chlorotrifluoroethylene and of trifluoroethylene from 1,1,2-trichloro-1,2,2-trifluoroethane and catalytic composition used in this process

(30) Priorité: 25.01.1991 BE 9100066
(43) Date de publication de la demande: 29.07.1992
(73) Titulaire: SOLVAY (Société Anonyme), B-1050 Bruxelles (BE)
(72) Inventeur: Lerot, Luc, B-1200 Bruxelles (BE); Wilmet, Vincent, B-1301 Bierges (BE); Pirotton, Joseph, B-1030 Bruxelles (BE)
(74) Mandataire: Jacques, Philippe

(56) Documents cités:
- EP-A- 0 253 410
- EP-A- 0 355 907
- EP-A- 0 459 463
- EP-A- 0 485 246
- WO-A-89/00452
- DE-A- 3 804 265
- DE-B- 1 044 800
- US-A- 3 505 417

## Description

La présente invention concerne un procédé pour la préparation de chlorotrifluoroéthylène et de trifluoroéthylène au départ de 1,1,2-trichloro-1,2,2-trifluoroéthane, ainsi qu'une composition catalytique utilisée dans ce procédé.

Le chlorotrifluoroéthylène et le trifluoroéthylène sont des monomères intéressants pour la fabrication de nombreux polymères, homo- et copolymères, fluorés et chlorofluorés.

La demande de brevet DE-B-1044800 décrit la préparation du chlorotrifluoroéthylène par réaction entre du 1,1,2-trichloro-1,2,2-trifluoroéthane et de l'hydrogène en présence d'un catalyseur constitué de chlorure de cuivre déposé sur charbon actif.

Dans la demande de brevet européen EP-A-0 253 410 au nom de AUSIMONT, on décrit la préparation de fluoroéthylènes et de chlorofluoroéthylènes par réaction en phase gazeuse de chlorofluoroéthanes en présence d'hydrogène à l'intervention de catalyseurs supportés sur carbone constitués de palladium, de nickel, de chrome, de cobalt, de platine, de cuivre ou de leurs mélanges, la préférence étant donnée au palladium et au nickel. Aucune composition catalytique comprenant plusieurs métaux n'y est toutefois décrite. D'autre part, le seul exemple où on décrit l'application de ce procédé au 1,1,2-trichloro-1,2,2-trifluoroéthane et qui concerne l'utilisation d'un catalyseur constitué de palladium supporté sur charbon montre une activité catalytique et une sélectivité en chlorotrifluoroéthylène et en trifluoroéthylène relativement faible, pratiquement 50 % des produits de la réaction étant constitués de trifluoroéthane, sous-produit par hydrogénation.

La présente invention a pour but de procurer un procédé pour la préparation de chlorotrifluoroéthylène et de trifluoroéthylène avec un taux de transformation de 1,1,2-trichloro-1,2,2-trifluoroéthane et une sélectivité en chlorotrifluoroéthylène et en trifluoroéthylène notablement améliorés.

A cet effet, l'invention concerne un procédé pour la préparation de chlorotrifluoroéthylène et de trifluoroéthylène par réaction en phase gazeuse de 1,1,2-trichloro-1,2,2-trifluoroéthane en présence d'hydrogène et d'une composition catalytique comprenant un support à base de carbone sur lequel sont déposés du cuivre et au moins un métal du groupe VIII du tableau périodique des éléments.

La demande de brevet européen EP-A-0485246, non publiée à la date de dépôt de la présente demande, décrit un procédé similaire, mettant en oeuvre un catalyseur contenant de 1 à 20 %. en poids de cuivre et de 0,1 à 10 % en poids d'un métal du groupe VIII.

Le procédé selon l'invention offre d'excellents résultats se traduisant notamment par un taux de transformation de 1,1,2-trichloro-1,2,2-trifluoroéthane généralement supérieur à 75 % en poids. Il offre également une excellente sélectivité en chlorotrifluoroéthylène et en trifluoroéthylène, généralement supérieure à 90 % en poids. Il permet enfin de moduler, en fonction des conditions de réaction, les proportions respectives de chlorotrifluoroéthylène et de trifluoroéthylène dans le mélange produit.

Un effet surprenant de la présente invention réside dans le fait que la mise en oeuvre d'une composition catalytique comprenant seulement du cuivre déposé sur carbone présente une activité catalytique insuffisante et que la mise en oeuvre d'une composition catalytique comprenant seulement du palladium déposé sur carbone présente une sélectivité en chlorotrifluoroéthylène et en trifluoroéthylène très faible, alors que le procédé selon l'invention procure à la fois un taux de transformation élevé du 1,1,2-trichloro-1,2,2-trifluoroéthane (c'est-à-dire une activité catalytique élevée) et une sélectivité élevée en chlorotrifluoroéthylène et en trifluoroéthylène (c'est-à-dire une production négligeable de sous-produits hydrogénés).

Parmi les métaux du groupe VIII du tableau périodique des éléments, utilisables seuls ou en mélange, on donne la préférence au ruthénium, au rhodium, à l'irridium, au platine et au palladium et plus particulièrement encore à l'irridium, au platine et au palladium. Les métaux du groupe VIII du tableau périodique des éléments tout particulièrement préférés sont le platine et le palladium.

Le procédé selon l'invention met en oeuvre une composition catalytique qui comprend au moins 0,05 % et, par ailleurs, pas plus de 10 % en poids de métal du groupe VIII du tableau périodique des éléments, par rapport au poids total de la composition catalytique. De préférence, elle comprend de 1 % à 5 % en poids de métal du groupe VIII du tableau périodique des éléments, par rapport au poids total de la composition catalytique.

Le procédé selon l'invention met en oeuvre une composition catalytique qui comprend au moins 0,1 % et, par ailleurs, pas plus de 50 % en poids de cuivre par rapport au poids total de la composition catalytique, à l'exception d'une teneur de 1 à 20 %. De préférence, on ne dépasse pas 30 % en poids de cuivre, par rapport au poids total de la composition catalytique.

En augmentant la quantité de cuivre mise en oeuvre, on peut améliorer la sélectivité en chlorotrifluoroéthylène et moduler ainsi les proportions respectives de chlorotrifluoroéthylène et de trifluoroéthylène produits. Pour ce faire, on préfère tout particulièrement travailler avec une concentration d'au moins 5 % en poids de cuivre par rapport au poids total de la composition catalytique.

Le rapport en poids entre le cuivre et le métal du groupe VIII du tableau périodique des éléments dans la composition catalytique peut varier dans de larges limites. Le plus souvent, il est d'au moins 0,1 sans excéder 20. On travaille avantageusement avec un rapport d'au moins 0,4. De préférence, on ne dépasse pas un rapport de 10.

Le cuivre et le métal (ou les métaux) du groupe VIII du tableau périodique des éléments sont généralement mis en oeuvre dans le procédé selon l'invention sous forme de composés organiques ou inorganiques. Comme composés organiques utilisables, on peut mentionner les carboxylates, les alcoolates et les acétylacétonates, ayant une chaîne alkyle qui contient de 1 à 10 atomes de carbone. Comme composés inorganiques utilisables, on peut mentionner les halogénures, les hydroxydes et les nitrates et plus particulièrement, les halogénures et les hydroxydes. De manière avantageuse, on choisit les chlorures, fluorures et hydroxydes. On obtient d'excellents résultats avec les chlorures.

Le support à base de carbone est généralement constitué de charbon actif ayant un important volume poreux. Le plus souvent, ce volume poreux est compris entre 0,1 et 2 cm³/g. Il est de préférence compris entre 0,2 et 1 cm³/g.

La surface spécifique du support à base de carbone est généralement comprise entre 10 et 1.500 m²/g.

Le procédé selon l'invention met en oeuvre une composition catalytique qui peut être obtenue par imprégnation du support avec les solutions de composés métalliques.L'imprégnation du support peut être réalisée par n'importe quelle méthode. En pratique, elle peut être obtenue par la technique dite du "volume poreux" (imprégnation dite "sèche") ou par la technique du "volume excédentaire" (imprégnation dite "humide"). De telles méthodes sont décrites dans la littérature, en particulier par Charles N. Satterfield "Heterogeneous catalysis in practice", 1980, Mc Graw-Hill, New-York, spécialement p. 82 et 83, qui est incorporé à la présente demande par référence.

Les solutions d'imprégnation peuvent être aqueuses ou organiques. De préférence, on met en oeuvre une solution aqueuse ou alcoolique.

L'ordre d'imprégnation du support n'est pas critique. L'imprégnation peut donc être réalisée d'abord avec une solution comprenant le cuivre ou encore d'abord avec une solution comprenant au moins un métal du groupe VIII du tableau périodique des éléments, ou simultanément avec ces deux solutions. De préférence, on imprègne d'abord avec une solution comprenant le métal présent en quantité prépondérante.

Après imprégnation, le support peut être séché avant d'être introduit dans le réacteur proprement dit.

La composition catalytique ainsi obtenue peut être mise en oeuvre dans le procédé selon l'invention telle quelle ou peut être préalablement réduite soit par de l'hydrogène, soit par un mélange d'hydrogène avec un gaz inerte tel que l'hélium.

La température à laquelle s'effectue la réaction selon le procédé de l'invention se situe habituellement entre 80 et 600°C. De préférence, cette température se situe entre 120 et 400°C et plus particulièrement encore entre 200 et 300°C.

La pression sous laquelle est effectuée la réaction n'est pas critique en elle-même. Habituellement, on opère sous une pression comprise entre 1x10⁵ et 10x10⁵ pascals. De préférence, on retient une pression comprise entre 2x10⁵ et 5x10⁵ pascals.

Le rapport volumique entre l'hydrogène et le 1,1,2-trichloro-1,2,2-trifluoroéthane mis en oeuvre est généralement compris entre 0,25 et 20. De préférence, il est d'au moins 0,4 et ne dépasse pas 10. Suivant un mode de réalisation particulièrement préféré, ce rapport est compris entre 1 et 7. C'est ainsi qu'avec un rapport situé aux environs de 4, d'excellents résultats ont été obtenus qui se traduisent en particulier par une très grande stabilité de la composition catalytique.

Le temps de contact moyen entre la composition catalytique et les produits en réaction est généralement d'au moins 0,5 secondes et ne dépasse pas 10 secondes. De préférence, il est d'au moins 3 secondes et ne dépasse pas 7 secondes.

Le procédé selon l'invention peut être réalisé en présence d'un gaz inerte tel que par exemple l'hélium.

Le procédé selon l'invention peut être mis en oeuvre dans tous types de réacteurs. En particulier, il peut aussi bien être mis en oeuvre dans un réacteur à lit fixe que dans un réacteur à lit fluidisé.

L'invention concerne également une composition catalytique pour la préparation de chlorotrifluoroéthylène et de trifluoroéthylène par réaction en phase gazeuse de 1,1,2-trichloro-1,2,2-trifluoroéthane en présence d'hydrogène, laquelle comprend un support à base de carbone sur lequel sont déposés du cuivre et au moins un métal du groupe VIII du tableau périodique des éléments, dans les teneurs précisées à la revendication 8.

Les exemples non limitatifs qui suivent sont donnés dans le but d'illustrer l'invention. Les exemples 1 et 2 sont donnés à titre de référence. Les exemples 3 à 5 illustrent la présente invention.

### Exemple 1 (de référence) : Palladium sur carbone

### a) Préparation de la composition catalytique

Dans une ampoule de 50 cm³, on introduit 10 cm³, soit 3,7 g d'un support à base de carbone (C LURGI ASIV 420), d'une surface spécifique de 1.100 m²/g et d'un volume poreux de 0,6 cm³/g.

On chauffe l'ampoule sous vide (1 à 600 Pa) à 125°C pendant 2 heures en vue de sécher et de dégazer le support.

Après refroidissement, le support est imprégné sous vide à température ambiante par 2,5 cm³ d'une solution aqueuse à 10 % en volume d'acide chlorhydrique concentré comprenant 0,16 g de chlorure de palladium. On laisse l'imprégnation se poursuivre pendant 2 heures sous vide et environ 16 heures à pression atmosphérique à température ambiante.

On sèche ensuite durant 3 heures à la pression atmosphérique à 120°C.

La composition catalytique ainsi obtenue comprend 2,2 % en poids de palladium par rapport au poids total de la composition catalytique.

2 cm³ de cette composition catalytique sont introduits dans un réacteur constitué d'un tube métallique en inox long de 520 mm et d'un diamètre intérieur de 7,7 mm ; la composition catalytique est ensuite conditionnée 2 heures à 240 °C sous 3x10⁵ pascals au moyen d'un mélange d'hydrogène et d'hélium dans un rapport volumique 10/90 à un débit de 40 cm³/min.

### b) Hydrogénation du 1,1,2-trichloro-1,2,2-trifluoroéthane

Le réacteur est alimenté à raison de 0,011 mole/heure de 1,1,2-trichloro-1,2,2-trifluoroéthane et de 0,016 mole/heure d'hydrogène et de 0,08 mole/heure d'hélium à 240°C sous 3x10⁵ pascals. Le temps de séjour moyen est évalué à 4,8 s.

Après 10 heures de fonctionnement, le taux de transformation du 1,1,2-trichloro-1,2,2-trifluoroéthane est de 71 % en poids, la sélectivité en chlorotrifluoroéthylène est de 14 % et la sélectivité en trifluoroéthylène est de 42 %, en poids également.

### Exemple 2 (de référence) : Cuivre sur carbone

Une composition catalytique est préparée suivant le protocole décrit à l'exemple 1.

Le support utilisé est celui décrit à l'exemple 1.

La composition catalytique est dans ce cas préparée par une imprégnation de 3,54 g de support à base de carbone avec 2 cm³ d'une solution aqueuse comprenant 0,75 g de chlorure cuivrique. La composition catalytique comprend ainsi 8,2 % en poids de cuivre.

1 cm³ de cette composition catalytique est conditionné comme dans l'exemple 1, dans un réacteur identique.

Le réacteur est alimenté à raison de 0,022 mole/heure de 1,1,2-trichloro-1,2,2-trifluoroéthane et de 0,032 mole/heure d'hydrogène à 240 °C sous 3x10⁵ pascals. Le temps de séjour moyen est évalué à 4,8 s.

Après 10 heures de fonctionnement, le taux de transformation du 1,1,2-trichloro-1,2,2-trifluoroéthane est de 12 %, la sélectivité en chlorotrifluoroéthylène est de 60 % et la sélectivité en trifluoroéthylène est de 3 %. Après 20 heures de fonctionnement, le taux de conversion n'est plus que de 8 %.

### Exemple 3 : Cuivre et palladium sur carbone

Une composition catalytique est préparée suivant le protocole décrit à l'exemple 1.

Le support utilisé est celui décrit à l'exemple 1.

La composition catalytique est dans ce cas préparée par une imprégnation de 4 g de support à base de carbone. Le support est d'abord imprégné avec 2,5 cm³ d'une solution aqueuse comprenant 0,91 g de chlorure cuivrique. Ensuite, le support est imprégné avec 2,5 cm³ d'une solution aqueuse à 10 % en volume d'acide chlorhydrique concentré comprenant 0,16 g de chlorure de palladium.

La composition catalytique ainsi obtenue comprend 8,4 % en poids de cuivre et 1,9 % en poids de palladium par rapport au poids total de la composition catalytique.

1 cm³ de cette composition catalytique est conditionné comme dans l'exemple 1, dans un réacteur identique.

Le réacteur est alimenté à raison de 0,022 mole/heure de 1,1,2-trichloro-1,2,2-trifluoroéthane et de 0,032 mole/heure d'hydrogène à 240°C sous 3x10⁵ pascals. Le temps de séjour moyen est évalué à 4,8 s.

Après 10 heures de fonctionnement, le taux de transformation du 1,1,2-trichloro-1,2,2-trifluoroéthane est de 78 % en poids, la sélectivité en chlorotrifluoroéthylène est de 87 % et la sélectivité en trifluoroéthylène est de 8 %, en poids également.

### Exemple 4 : Cuivre et palladium sur carbone

Une composition catalytique est préparée suivant le protocole décrit à l'exemple 1.

Le support utilisé est celui décrit à l'exemple 1.

La composition catalytique est dans ce cas préparée par une imprégnation de 3,76 g de support à base de carbone. Le support est d'abord imprégné avec 2,5 cm³ d'une solution aqueuse comprenant 0,91 g de chlorure cuivrique. Il est ensuite imprégné avec 2,5 cm³ d'une solution aqueuse à 10 % en volume d'acide chlorhydrique concentré comprenant 0,16 g de chlorure de palladium.

La composition catalytique ainsi obtenue comprend 8,9 % en poids de cuivre et 2,0 % en poids de palladium par rapport au poids total de la composition catalytique.

3 cm³ de cette composition catalytique sont conditionnés comme dans l'exemple 1, dans un réacteur identique.

Le réacteur est alimenté à raison de 0,022 mole/heure de 1,1,2-trichloro-1,2,2-trifluoroéthane et de 0,086 mole/heure d'hydrogène à 240°C sous 2x10⁵ pascals. Le temps de séjour moyen est évalué à 4,8 s.

Après 10 heures de fonctionnement, le taux de transformation du 1,1,2-trichloro-1,2,2-trifluoroéthane est de 100 % en poids, la sélectivité en chlorotrifluoroéthylène est de 83 % et la sélectivité en trifluoroéthylène est de 16 %, en poids également. Après 300 heures de fonctionnement, le taux de transformation du 1,1,2-trichloro-1,2,2-trifluoroéthane est encore de 95 % en poids, les sélectivités en chlorotrifluoroéthylène et en trifluoroéthylène étant inchangées.

### Exemple 5 : Cuivre et platine sur carbone

Une composition catalytique est préparée suivant le protocole décrit à l'exemple 1.

Le support utilisé est celui décrit à l'exemple 1.

La composition catalytique est dans ce cas préparée par une imprégnation de 3,67 g de support à base de carbone. Le support est d'abord imprégné avec 2,5 cm³ d'une solution aqueuse comprenant 0,76 g de chlorure cuivrique. Il est ensuite imprégné avec 2,5 cm³ d'une solution aqueuse à 10 % en volume d'acide chlorhydrique concentré comprenant 0,14 g de chlorure de platine.

La composition catalytique ainsi obtenue comprend 7,9 % en poids de cuivre et 1,4 % en poids de platine par rapport au poids total de la composition catalytique.

1 cm³ de cette composition catalytique est conditionné comme dans l'exemple 1, dans un réacteur identique.

Le réacteur est alimenté à raison de 0,022 mole/heure de 1,1,2-trichloro-1,2,2-trifluoroéthane et de 0.032 mole/heure d'hydrogène à 240°C sous 3x10⁵ pascals. Le temps de séjour moyen est évalué à 4,8 s.

Après 10 heures de fonctionnement, le taux de transformation du 1,1,2-trichloro-1,2,2-trifluoroéthane est de 95 % en poids, la sélectivité en chlorotrifluoroéthylène est de 90 % et la sélectivité en trifluoroéthylène est de 8 %, en poids également.

La comparaison des résultats des exemples 2, 3 et 4 selon l'invention avec ceux de l'exemple 1 de référence illustre l'amélioration substantielle des taux de transformation du 1,1,2-trichloro-1,2,2-trifluoroéthane et de la sélectivité en chlorotrifluoroéthylène et en trifluoroéthylène que procurent le procédé et la composition catalytique selon l'invention.

## Revendications

1. Procédé pour la préparation de chlorotrifluoroéthylène et de trifluoroéthylène par réaction en phase gazeuse de 1,1,2-trichloro-1,2,2-trifluoroéthane en présence d'hydrogène et d'une composition catalytique, caractérisé en ce que la composition catalytique comprend un support à base de carbone sur lequel sont déposés du cuivre et au moins un métal du groupe VIII du tableau périodique des éléments et présente une teneur en cuivre de 1 à 50 % en poids, à l'exception d'une teneur de 1 à 20 %, et une teneur en métal du Groupe VIII de 0,05 à 10 % en poids, par rapport au poids total de la composition catalytique.

2. Procédé selon la revendication 1, caractérisé en ce que le métal du groupe VIII du tableau périodique des éléments est choisi parmi le ruthénium, le rhodium, l'irridium, le platine, le palladium et leurs mélanges.

3. Procédé selon la revendication 2, caractérisé en ce que le métal du groupe VIII du tableau périodique des éléments est choisi parmi l'irridium, le platine , le palladium et leurs mélanges.

4. Procédé selon la revendication 3, caractérisé en ce que le métal du groupe VIII du tableau périodique des éléments est choisi parmi le platine, le palladium et leurs mélanges.

5. Procédé selon une quelconque des revendications 1 à 4, caractérisé en ce que la composition catalytique comprend de 1 à 5 % en poids de métal du groupe VIII du tableau périodique des éléments par rapport au poids total de la composition catalytique.

6. Procédé selon la revendication 1, caractérisé en ce que la composition catalytique comprend 30 % en poids ou moins de cuivre par rapport au poids total de la composition catalytique.

7. Procédé selon la revendication 1, caractérisé en ce que le rapport volumique entre l'hydrogène et le 1,1,2-trichloro-1,2,2-trifluoroéthane mis en oeuvre est compris entre 1 et 7.

8. Composition catalytique pour la préparation de chlorotrifluoroéthylène et de trifluoroéthylène par réaction en phase gazeuse de 1,1,2-trichloro-1,2,2-trifluoroéthane en présence d'hydrogène, caractérisée en ce qu'elle comprend un support à base de carbone sur lequel sont déposés du cuivre et au moins un métal du groupe VIII du tableau périodique des éléments et en ce qu'elle présente une teneur en cuivre de 1 à 50 % en poids, à l'exception d'une teneur de 1 à 20 %, et une teneur en métal du Groupe VIII de 0,05 à 10 % en poids, par rapport au poids total de la composition catalytique.

9. Procédé de préparation de la composition catalytique conforme à la revendication 8, selon lequel on imprègne un support à base de carbone d'abord avec une solution comprenant un métal du groupe VIII du tableau périodique des éléments et ensuite avec une solution comprenant du cuivre.

## Claims

1. Process for preparing chlorotrifluoroethylene and trifluoroethylene by reaction in gaseous phase of 1,1,2-trichloro-1,2,2-trifluoroethane in the presence of hydrogen and a catalytic composition, characterised in that the catalytic composition comprises a carbon-based support on which copper and at least one group VIII metal in the Periodic Table of elements are deposited and has a copper content from 1 to 50 weight percent, a content from 1 to 20 % being excepted, and a Group VIII metal content from 0.05 to 10 weight percent, relative to the total weight of the catalytic composition.

2. Process according to Claim 1, characterised in that the group VIII metal in the Periodic Table of elements is chosen from ruthenium, rhodium, iridium, platinum, palladium and their mixtures.

3. Process according to Claim 2, characterised in that the group VIII metal in the Periodic Table of elements is chosen from iridium, platinum, palladium and their mixtures.

4. Process according to Claim 3, characterized in that the group VIII metal in the Periodic Table of elements is chosen from platinum, palladium and their mixtures.

5. Process according to any of Claims 1 to 4, characterised in that the catalytic composition comprises from 1 to 5 % by weight of a group VIII metal in the Periodic Table of elements relative to the total weight of the catalytic composition.

6. Process according to Claim 1, characterised in that the catalytic composition comprises 30 % by weight or less of copper relative to the total weight of the catalytic composition.

7. Process according to Claim 1, characterised in that the volume ratio between the hydrogen and the 1,1,2-trichloro-1,2,2-trifluoroethane used is between 1 and 7.

8. Catalytic composition for preparing chlorotrifluoroethylene and trifluoroethylene by reaction in gaseous phase of 1,1,2-trichloro-1,2,2-trifluoroethane in the presence of hydrogen, characterised in that it comprises a carbon-based support on which copper and at least one group VIII metal in the Periodic Table of elements are deposited, having a copper content from 1 to 50 weight percent, a content from 1 to 20 % being excepted, and a Group VIII metal content from 0.05 to 10 weight percent, relative to the total weight of the catalytic composition.

9. Process for preparing the catalytic composition according to Claim 8, wherein a carbon-based support is first impregnated with a solution comprising a group VIII metal in the Periodic Table of the elements and thereafter with a solution comprising copper.

## Patentansprüche

1. Verfahren zur Herstellung von Chlortrifluorethylen und Trifluorethylen durch Reaktion in der Gasphase von 1,1,2-Trichlor-1,2,2-trifluorethan in Gegenwart von Wasserstoff und einer katalytischen Zusammensetzung, dadurch gekennzeichnet, daß die katalytische Zusammensetzung einen Träger auf der Basis von Kohlenstoff umfaßt, auf dem Kupfer und mindestens Bin Metall der Gruppe VIII des Periodensystems der Elemente abgeschieden sind, und bezogen auf das Gesamtgewicht der katalytischen Zusammensetzung einen Gehalt an Kupfer von 1 bis 50 Gew.-%, mit Ausnahme eines Gehalts von 1 bis 20%, und einen Gehalt an Metall der Gruppe VIII von 0,05 bis 10 Gew-% aufweist.

2. Verfahren gemäß Anspruch 1, dadurch gekennzeichnet, daß das Metall dar Gruppe VIII des Periodensystems der Elemente unter Ruthenium, Rhodium, Iridium, Platin, Palladium und ihren Gemischen ausgewählt ist.

3. Verfahren gemäß Anspruch 2, dadurch gekennzeichnet, daß das Metall der Gruppe VIII des Periodensystems der Elemente unter Iridium, Platin, Palladium und ihren Germischen ausgewählt ist.

4. Verfahren gemäß Anspruch 3, dadurch gekennzeichnet, daß das Metall der Gruppe VIII des Periodensystems der Elemente unter Platin, Palladium und ihren Gemischen ausgewählt ist.

5. Verfahren gemäß einem der Ansprüche 1 bis 4, dadurch gekennzeichnet, daß die katalytische Zusammensetzung bezogen auf das Gesamtgewicht der katalytischen Zusammensetzung 1 bis 5 Gew.-% Metall der Gruppe VIII des Periodensystems der Elemente umfaßt.

6. Verfahren gemäß Anspruch 1, dadurch gekennzeichnet, daß die katalytische Zusammensetzung bezogen auf das Gesamtgewicht der katalytischen Zusammensetzung 30 Gew.-% oder weniger Kupfer umfaßt.

7. Verfahren gemäß Anspruch 1, dadurch gekennzeichnet daß das eingesetzte Volumenverhältnis zwischen Wasserstoff und 1,1,2-Trichlor-1,2,2-trifluorethan zwischen 1 und 7 liegt.

8. Katalytische Zusammensetzung zur Herstellung von Chlortrifluorethylen und Trifluorethylen durch Reaktion in der Gasphase von 1,1,2-Trichlor-1,2,2-trifluorethan in Gegenwart von Wasserstoff, dadurch gekennzeichnet, daß sie einen Träger auf der Basis von Kohlenstoff, auf dem Kupfer und wenigstens ein Metall der Gruppe VIII des Periodensystems der Elemente abgeschieden sind, umfaßt und dadurch, daß sie bezogen auf das Gesamtgewicht der katalytischen Zusammensetzung einen Gehalt an Kupfer von 1 bis 50 Gew.-%, mit Ausnahme eines Gehalts von 1 bis 20%, und einen Gehalt an Metall der Gruppe VIII von 0,05 bis 10 Gew.-% aufweist.

9. Verfahren zur Herstellung der katalytischen Zusammensetzung gemäß Anspruch 8, gemäß dem man einen Träger auf der Basis von Kohlenstoff zuerst mit einer Lösung, die ein Metall der Gruppe VIII des Periodensystems der Elemente umfaßt, und dann mit einer Lösung, die Kupfer umfaßt, tränkt.
